# EUROPEAN PATENT APPLICATION

(11) **EP 2 600 265 A1**
(43) Date of publication of application: **05.06.2013**
(21) Application number: 11191832.2
(22) Date of filing: 02.12.2011
(51) Int. Cl.: G06F 19/00, A61H 31/00

(54) **A device for performing diagnostics and/or therapy and related kit, system, computer program, methods and computer readable mediums**

(71) Applicant: Zotz, Rainer, 54634 Bitburg (DE)
(72) Inventor: Zotz, Rainer, 54634 Bitburg (DE)
(74) Representative: KIPA AB

(57) **Abstract**

The disclosure relates to a device for performing diagnostics and/or therapy and related kit, system, computer program, methods and computer readable mediums. The device of this disclosure has the advantages that the device does not need access to the pericardium and that the whole system including kit and device can be removed after diagnostics/therapy. A device for performing diagnostics and/or therapy of an organ in the thoracic cavity, comprising an organ support (10), which organ support (10) is devised to be arranged exterior to an organ, and comprises: a flexible structure (14), such as a fabric, net, coil, bag or mesh, which is configured to cover a region of said organ, said region comprising at least the surface at an apex of said organ; at least one actuator (16), such as a motor, for actuating a movement of said flexible structure (14) for said therapy, and optionally at least one transducer (18), is provided.

## Description

### Field of the Invention

This disclosure pertains in general to the field of diagnosis and/or therapy of an organ, such as a heart. More particularly, the disclosure relates to a device for performing diagnostics and/or therapy of an organ, such as a heart and related kit, system, computer program, methods and computer readable mediums.

### Background of the Invention

Different cardiac harnesses are known. One example of such a cardiac harness is disclosed in US Patent 6,569,082 B1. The apparatus disclosed in this document is used for cardiac restraint and to implement the function of the apparatus, the pericardium needs to be accessed.

Accessing the pericardium often means that some damage is done to the pericardium.

Thus, a device which does not need access to the pericardium would be advantageous, since then a less invasive procedure could be performed.

Furthermore, since prior art devices need access to the pericardium, they are not normally removed after insertion.

Thus, a system, including kit and device, which can be removed after diagnosis and/or therapy would be advantageous.

### Summary of the Invention

Accordingly, embodiments of the present disclosure preferably seek to mitigate, alleviate or eliminate one or more deficiencies, disadvantages or issues in the art, such as the above-identified, singly or in any combination by providing a device for performing diagnostics and/or therapy and related kit, computer program, methods and computer readable mediums according to the appended patent claims.

A disadvantage with the prior art method disclosed in US Patent 6,569,082 B1 is that a sub-xiphoid incision has to be made. The present disclosure overcomes this and/or other disadvantages with prior art by providing a device, which can be inserted to a target site outside the pericardium, by the use of the epicardial access approach, which is an approach in which a device can be inserted via the left internal mammary puncture from the left arm, i.e. there is no need for any sub-xiphoid incision.

According to one aspect of the disclosure, a device for performing diagnostics and/or therapy of an organ in the thoracic cavity is provided, which comprises an organ support, which organ support is devised to be arranged exterior to an organ, and comprises: a flexible structure, such as a fabric, net, coil, bag or mesh, which is configured to cover a region of the organ, the region comprising at least the surface at an apex of the organ; at least one actuator, such as a motor, for actuating a movement of the flexible structure for the therapy, and optionally at least one transducer.

According to another aspect of the disclosure, a method of performing diagnostics of an organ in the thoracic cavity is provided, which comprises receiving at least one measurement signal from at least one transducer at an internal control unit, transmitting the at least one measurement signal from the internal control unit to a computer, computing in the computer a diagnosis of an organ based on the at least one measurement signal, searching in the computer a database for therapy methods corresponding to the diagnosis, computing a score for each therapy method according to a criteria, selecting a therapy method with the highest score as a best matched therapy method, comparing the score of the best matched therapy method to a threshold value and displaying the best matched therapy method with corresponding score if the score of the best matched therapy method is above the threshold value, and otherwise not displaying any therapy method.

According to yet another aspect of the disclosure, a computer-readable medium having embodied thereon a computer program for processing by a computer, which computer program comprises a code segment for receiving at least one measurement signal, a code segment for computing a diagnosis of an organ based on the at least one measurement signal, a code segment for searching a database for therapy methods corresponding to the diagnosis, a code segment for computing a score for each therapy method according to a criteria, a code segment for selecting a therapy method with the highest score as a best matched therapy method, a code segment for comparing the score of the best matched therapy method to a threshold value, a code segment for displaying the best matched therapy method with corresponding score if the score of the best matched therapy method is above the threshold value, and otherwise not displaying any therapy method, is provided.

According to yet another aspect of the disclosure, a computer program enabling carrying out the above-mentioned method is provided.

According to yet another aspect of the disclosure, a non-transitory computer-readable storage medium encoded with programming instructions, the storage medium being loaded into a computerized control system of an apparatus for performing diagnostics of an organ in the thoracic cavity, and the programming instructions causing the computerized control unit to control the apparatus during operation by: receiving at least one measurement signal from at least one transducer at an internal control unit, transmitting the at least one measurement signal from the internal control unit to a computer, computing in the computer a diagnosis of an organ based on the at least one measurement signal, searching in the computer a database for therapy methods corresponding to the diagnosis, computing a score for each therapy method according to a criteria, selecting a therapy method with the highest score as a best matched therapy method, comparing the score of the best matched therapy method to a threshold value and displaying the best matched therapy method with corresponding score if the score of the best matched therapy method is above the threshold value, and otherwise not displaying any therapy method, is provided.

According to yet another aspect of the disclosure, a kit for delivery of a device is provided, which comprises a flexible protecting catheter for bringing the flexible structure of the removable device to a target site in a body, exterior to the pericardial sack of a heart, a guide wire for guiding the flexible protecting catheter to the target site in the body, exterior to the pericardial sack of the heart, a tool for facilitate lifting of the heart, a surgical tool for putting the flexible structure around the pericardial sack of the heart, so that the flexible structure surrounds part of the heart and a surgical tool, such as three-arm-pliers or four-arm-pliers, for bringing the flexible structure back to its collapsed state.

According to yet another aspect of the disclosure, a method for delivery of a device is provided, which comprises gaining epicardial access through the use of the epicardial access approach via the left internal mammary puncture from the left arm, bringing the flexible structure into a collapsed state, placing the collapsed flexible structure inside a protecting catheter, delivering the collapsed flexible structure with the protecting catheter to a target site exterior to the pericardial sack and sliding the flexible structure around the pericardial sack of a heart, so that the flexible structure surrounds part of the heart.

According to a further aspect of the disclosure, a method of temporarily treatment of an organ in a thoracic cavity is provided, which comprises providing a device, and activating a therapy program, wherein activating is regulated in a control loop based on a control signal based on sensor measurement.

According to one aspect of the disclosure, a system, comprising a kit and a device is provided.

Further embodiments of the disclosure are defined in the dependent claims, wherein features for the second and subsequent aspects of the disclosure are as for the first aspect mutatis mutandis.

Some embodiments of the disclosure provide for an organ support/heart support, which is provided outside the pericardium, and thus a less invasive procedure can be performed.

Some embodiments of the disclosure also provide for assisting in breathing via diaphragm activation.

Some embodiments of the disclosure also provide for that treatment of hardening of coronary artery, i.e. arteriosclerosis in the coronary artery can be performed.

Some embodiments of the disclosure also provide for obtaining measurements with an improved quality, since the thorax is no longer an obstruction for measurements of an organ.

Some embodiments of the disclosure also provide for enabling four dimensional imaging.

Some embodiments of the disclosure also provide for enabling the viewing of real-time movement realistically.

Some embodiments of the disclosure also enable use of four dimensional technologies, which adds motion to the overall picture, allowing a physician to view such kinetic phenomena as blood flow and muscle contractions.

Some embodiments of the disclosure also provide for enabling the use of nuclear medicine, which can tell how well an organ works, rather than provide images of the organ.

Some embodiments of the disclosure facilitate transfer of signals related to magnetic resonance through the skin of a patient.

Some embodiments of the disclosure enable magnetic resonance tomography or magnetic resonance imaging inside a body.

Some embodiments of the disclosure enable local interior magnetic resonance imaging.

Some embodiments of the disclosure also provide for that the function of left ventricle assist device can be implemented.

Some embodiments of the disclosure enable the use of a motor as a sensor.

Some embodiments of the disclosure also provide for a wireless transfer of energy.

Some embodiments of the disclosure also provide for that the battery does not need to be replaced often or at all.

Some embodiments of the disclosure also provide for a transfer of signals for controlling an actuator.

Some embodiments of the disclosure also provide for protection of integrity and authenticity of transmitted radio frequency signals.

Some embodiments of the disclosure also provide for aiding a physician in selecting a therapy method, and thereby also in treating patients.

Some embodiments of the disclosure also provide for a removable device and a removable system including kit and device, i.e. device and system do not need to be permanent, but can be removed after therapy/diagnosis/inspection.

Some embodiments of the disclosure enable the removal of a flexible structure through a body opening after use.

Some embodiments of the disclosure also provide for substantially lower complication prospects than conventional procedures, through the use of the epicardial access method.

Some embodiments of the disclosure also provide for a substantially lower patient risk, through the use of the epicardial access method.

Some embodiments of the disclosure also provide for a very good signal-to-noise ratio, since the thorax is no longer an obstruction for the measurements

Some embodiments of the disclosure also provide for an automatic inspection independent reproducible analysis of congenital heart defects, left ventricle function, perfusion, diameter, contractility, and/or strain-4D

Some embodiments of the disclosure also provide for an automatic quantification of perfusion and coronary stenosis.

Some embodiments of the disclosure also provide for diagnostics, which is better, more reliable and more cost-efficient than invasive catheter diagnostics, since the device used is removable, the procedure performed is less invasive, a better quality measurement is provided, and since it is possible to leave the device inside a body for a longer period of time.

Some embodiments of the disclosure also provide for the possibility of analysing measurements in four dimensions.

Some embodiments of the disclosure also provide for that the use of X-rays is avoided.

Some embodiments of the disclosure also provide for that an analysis can be performed on raw data instead of processed data.

Some embodiments of the disclosure also provide for control of intravenous interventions.

Some embodiments of the disclosure also provide for that references to literature or links to Internet, proposals for diagnosis, and/or treatment proposals can be given to a physician in a computer.

Some embodiments of the disclosure also provide for high quality images by the use of ultrasound tomography.

Some embodiments of the disclosure also enable the lifting of a heart with a spoon-formed controllable catheter, in order to enable sliding a flexible structure around a heart.

Some embodiments of the disclosure also provide for direct energy transportation.

It should be emphasized that the term "comprises/comprising" when used in this specification is taken to specify the presence of stated features, integers, steps or components but does not preclude the presence or addition of one or more other features, integers, steps, components or groups thereof.

### Brief Description of the Drawings

These and other aspects, features and advantages of which embodiments of the disclosure are capable of will be apparent and elucidated from the following description of embodiments of the present disclosure, reference being made to the accompanying drawings, in which
Fig. 1 depicts a heart surrounded by a flexible structure of a device for performing diagnostics and/or therapy of an organ in the thoracic cavity;
Fig. 2 depicts a device for performing diagnostics and/or therapy of an organ in the thoracic cavity, with a flexible structure surrounding the heart, and with electronics for transferring measurement signals;
Fig. 3 shows electronic parts of a device for measurements and transmitting data;
Fig. 4 shows different steps of a method for performing diagnostics of an organ in the thoracic cavity;
Fig. 5 shows code segments of a computer program for performing diagnostics of an organ in the thoracic cavity;
Fig. 6 is a front view of a patient and depicts a spoon-shaped catheter for lifting a heart;
Fig. 7 depicts a step of a method of delivering a device for performing diagnostics and/or therapy of an organ in the thoracic cavity;
Fig. 8 depicts another step of a method of delivering a device for performing diagnostics and/or therapy of an organ in the thoracic cavity;
Fig. 9 depicts the flexible structure 14 in its collapsed state;
Fig. 10 depicts the flexible structure 14 in its expanded state;
Fig. 11a is a front view of a patient with a device;
Fig. 11b shows a heart partly surrounded by a flexible structure, when the heart is in an expanded state, connected to a motor;
Fig. 11c shows a heart partly surrounded by a flexible structure, when the heart is in a contracted state, connected to a motor;
Fig. 12 shows the pericardial sack substantially covered by the flexible structure 14;
Fig. 13 shows the pericardial sack substantially covered by the flexible structure 14;
Fig. 14 shows a surgical tool for removing a flexible structure from a heart; and
Fig. 15 shows a flexible structure 14.

### Description of embodiments

Specific embodiments of the disclosure now will be described with reference to the accompanying drawings. This disclosure may, however, be embodied in many different forms and should not be construed as limited to the embodiments set forth herein; rather, these embodiments are provided so that this disclosure will be thorough and complete, and will fully convey the scope of the disclosure to those skilled in the art. The terminology used in the detailed description of the embodiments illustrated in the accompanying drawings is not intended to be limiting of the disclosure. In the drawings, like numbers refer to like elements.

The following description focuses on an embodiment of the present disclosure applicable to an organ and in particular to a heart. However, it will be appreciated that the disclosure is not limited to this application but may be applied to many other organs including for example a lung.

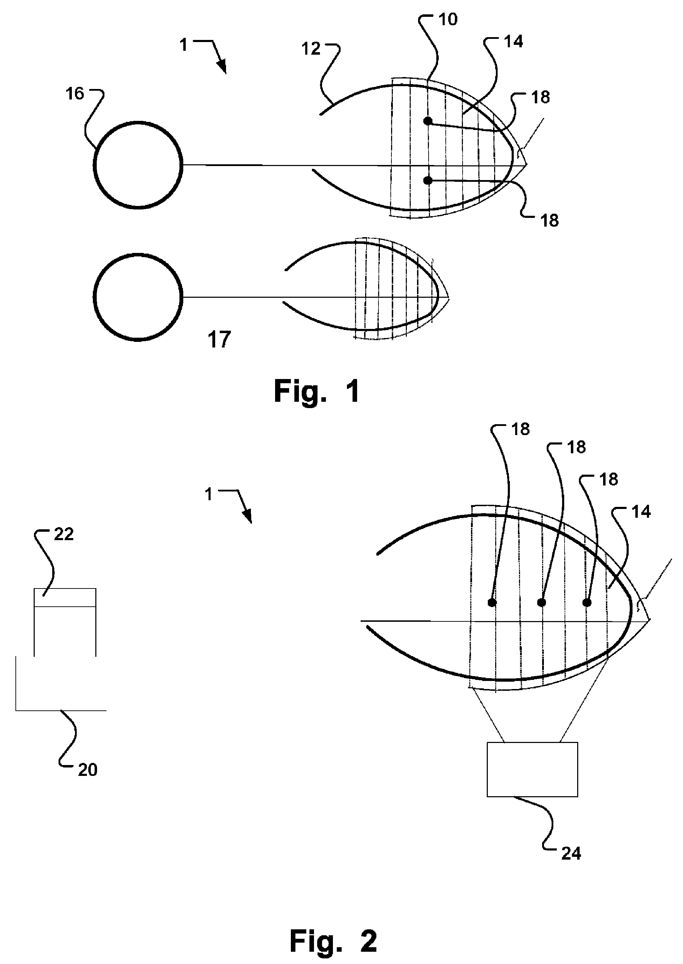

In an embodiment of the disclosure according to Fig. 1, a device 1 is provided with an organ support 10. The organ support 10 is devised to be arranged exterior to an organ, such as a heart, and comprises a flexible structure 14. The flexible structure 14 may be a fabric, a net, a coil or a mesh and it is configured to cover a region of the organ. The flexible structure 14 may be a hollow structure, having the shape of a sock. The region covered by the flexible structure 14 comprises the surface at an apex of the organ. Such an apex could be the lowest point of the organ or it could be the highest point of the organ. It could also be any other apex of the organ, such as the left-most point, the right-most point, the point furthest to the front, or the point furthest to the rear. The region covered by the flexible structure 14 may also be the whole organ. The organ support 10 further comprises at least one actuator 16 for actuating a movement of the flexible structure 14 for performing therapy of the organ. If the organ is a heart, the therapy may be performed inside and/or outside the pericardium. The actuator may be one or several motors, one or several pneumatic actuators or one or several hydraulic actuators, such as a pump. If the actuator 16 is a pneumatic or hydraulic actuator, it is preferably located exterior to the body. By implementing the device 1 this way, it will function as a left ventricle assist device.

The actuator may actuate a movement of the flexible structure 14, by pulling, pushing or spinning a connecting element 19. The connecting element 19 will then affect the flexible structure 14, so that the surface of the flexible structure 14 will become smaller or larger. Thereby an organ, such as a heart, can be assisted in its natural contractions and/or its natural expansions. Furthermore, the diastolic function may be augmented. Then during the diastole, the heart is actively dilated. Instruments used for augmentation can be at least one suction cup and/or any instrument, which allows the epicardium to be attached to it or hooked on to it. Along with instruments used for augmentation, other instruments may be used, and placed inside the heart and/or the lung. The organ support 10 may optionally comprise at least one transducer 18. Examples of transducers comprised by the organ support 10 are sensors, such as temperature sensors, accelerometers, voltage sensors, potential sensors, current sensors, pH sensors or transceivers, such as ultrasound transmitters or ultrasound receivers. Some or all of the transducers may instead or as well be actuators, e.g. for performing therapy of the organ. In one embodiment, the transducers are adapted to measure alpha radiation, beta radiation and/or gamma radiation, thereby enabling the use of nuclear medicine. The transducers 18 are preferably integrated with the flexible structure 14. In Fig. 1, a situation 17, where the device 1 is depicted, when the organ is assisted in its natural contracting, is shown. In one embodiment, the flexible structure 14 is located so closely to the organ, that there is no free space between the flexible structure 14 and the organ, i.e. some kind of vacuum exists. This vacuum may contribute in assisting the organ expanding.

Fig. 2 shows a device for performing diagnostics and/or therapy of an organ in the thoracic cavity, with a flexible structure 14 surrounding a heart. In Fig. 2, the flexible structure 14 is connected to an internal control unit 24. The internal control unit 24 may be connected to at least one transducer 18, by wires or wirelessly, and obtain measurements from the at least one transducer 18. Furthermore, the internal control unit 24 may send signals to the flexible structure 14. These signals can be based on measurements obtained by the internal control unit 24 from transducers 18. The flexible structure 14 is in this embodiment formed as a coil or comprises an electromagnetic coil and thus comprises the function of an electromagnetic coil, so that signals can be sent from the internal control unit 24 to an external control unit or a computer 20, via the flexible structure 14 and an external electromagnetic coil 22. The signals sent may be measurement signals, such as measurement signals related to magnetic resonance imaging MRI or magnetic resonance tomography MRT. Another example of measurement signals is measurement signals related to an electrocardiogram, obtained by at least one transducer 18. By sending measurement signals related to electrocardiograms continuously, an analysis in four dimensions, including time and a three-dimensional space, can be performed. The signals may alternatively be control signals sent from the computer 20 or an external control unit to control the flexible structure 14 or any of the transducers 18. As an example, the control signals may control at least one ultrasound transducer 18 to provide therapy to an organ, such as a heart. In one embodiment, a group of ultrasound transducers 18 are used for therapy. The ultrasound transducers 18 may for the therapy utilize pure wave technology. As an example, the output of the transducers for therapy may be pure sine waves. In order to achieve these pure sine waves, the control signals may as well be pure sine waves.

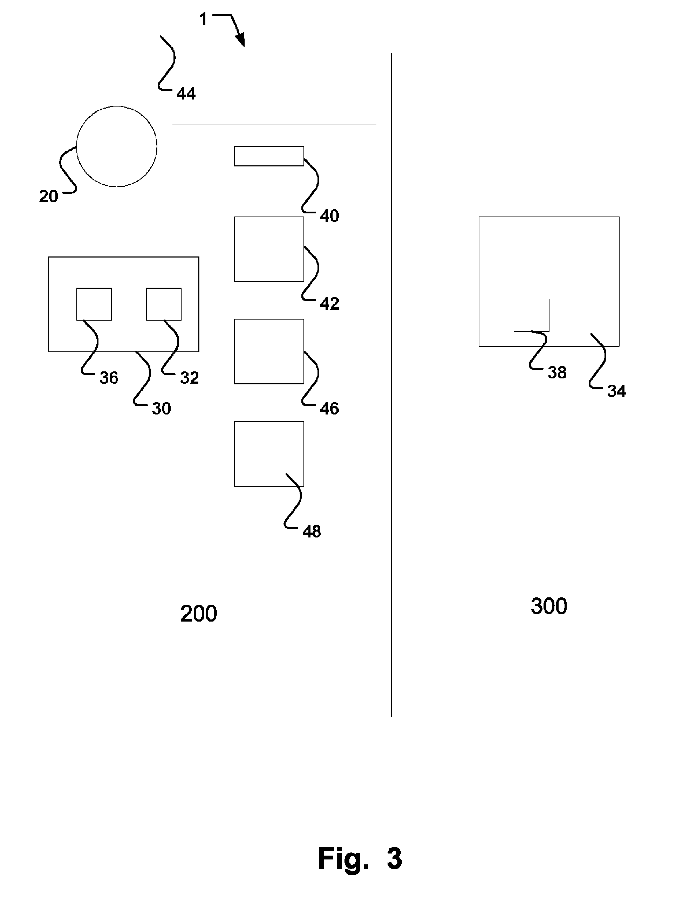

In an embodiment according to Fig. 3, the device 1 is provided with electronics for measurements and for transmitting data. In this embodiment, the actuator 16, which may be a motor, is located in the interior 200 of a body. The actuator 16 is provided with a component 40 arranged at a voltage supply 44 of the actuator 16. The component 40 may for example be a resistor and be connected to the electrical wires for supplying power in series or in parallel with the wires. Other examples of components that can be used are hall sensors, current sensors, temperature sensors for measuring the temperature of the actuator 16, infrared sensors or infrared temperature sensors. A measuring device 42 is connected to the component 40 for measuring a value related to the component 40. This measuring device 42 is thereby sensing a load of the actuator. For example, the measuring device 42 is arranged to sense a current supplied to the actuator 16 or arranged to sense a voltage supplied to the actuator 16. A comparison unit 46 is connected to the measuring device 42 for making a comparison between the value related to the component 40 and at least one expected value. There is also a decision unit 48, connected to the comparison unit 46 for determining a condition of a heart based on the comparison. In this embodiment, the actuator 16, which may be a motor, is acting as a sensor. The embodiment is directed to determining a condition of a heart. However, in another embodiment a condition of another organ, such as a lung, can be determined. In this embodiment, the flexible structure 14 is configured to cover at least an apex of the lung and preferably the whole lung. Furthermore, in this embodiment, the thorax may be moved in three dimensions from the inside and the main muscle, the diaphragm, can be stimulated electrically. Thereby, a normal pressure ventilation would be achieved as opposed to a high pressure ventilation, which is normally used in prior art, e.g. during tracheal intubation. Thus, the lung would be assisted in diaphragmatic breathing. This assisting in diaphragmatic breathing comprises an internal movement of the whole thorax, with or without the diaphragm, so that negative pressure breathing can be performed. Furthermore, the diagnosis and/or therapy of the lung may be performed simultaneously with diagnosis and/or therapy of the heart.

Fig. 3 also shows that the actuator 16 may be provided with an internal power supply 30. The internal power supply comprises a battery 32, and can be recharged from an external power supply 34, located exterior 300 to the body, through a wireless energy transfer. By transferring energy wirelessly, the battery 32 does not need to be replaced as often or at all. The internal power supply 30 may further comprise a radio frequency transceiver 36. The external power supply 34 may also comprise a radio frequency transceiver 38 and the radio frequency transceivers 36, 38 can be adapted to perform the wireless energy transfer. In one embodiment, the radio frequency transceivers 36, 38 are also adapted to transfer radio signals related to measurements, such as measurements obtained by at least one transducer 18. In another embodiment, the radio frequency transceivers 36, 38 are also adapted to transfer radio signals related to control of the actuator 16. The radio signals may also be encrypted, in order to provide for protection of integrity and authenticity.

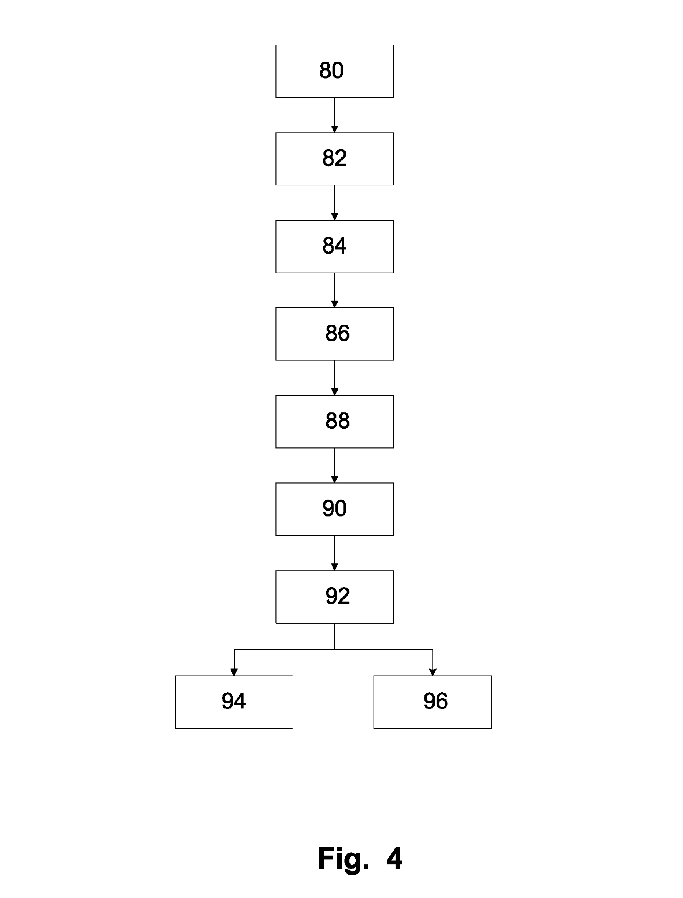

Fig. 4 shows different steps of a method for performing diagnostics of an organ in the thoracic cavity. In this method at least one measurement signal is received 80 from at least one transducer 18 at an internal control unit 24. Thereafter the at least one measurement signal is transmitted 82 from the internal control unit 24 to a computer 20. A diagnosis of an organ is computed 84 in the computer 20 based on the at least one measurement signal. If the organ is a heart, the diagnosis may include diagnosis outside and/or inside the pericardium. In the method, a database is searched 86 for therapy methods corresponding to the diagnosis. The search is performed in the computer 20. The database may be located in the computer 20 or remotely, and accessed over a network or the Internet. In the database a plurality of diagnoses are related to a plurality of therapy methods. Thereafter a score for each therapy method is computed 88 according to a criterion. The criterion used may be any suitable criterion, such as a criterion based on statistics or a criterion, in which each possible diagnosis only has one possible therapy method associated with it. Thereafter, a therapy method with the highest score is selected 90 as a best matched therapy method. The score of the best matched therapy method may then be compared 92 to a threshold value. The best matched therapy method is displayed 94 together with corresponding score if the score of the best matched therapy method is above the threshold value. Otherwise no therapy method is displayed 96. Optionally a message stating that no suitable therapy method was found may be displayed at step 96. As an alternative of displaying only the best matched therapy method, a plurality of therapy methods having a score, which is higher than a threshold value, may be displayed. By this method, a physician is aided in selecting a therapy method, and thereby also in treating patients. The method may also comprise an additional step of transmitting the at least one measurement signal from the internal control unit 24 to an external control unit, which external control unit conveys the signal to a computer 20. The transfer of signals from the interior of a body to the exterior of a body may be performed via internal and external electromagnetic coils. As another alternative, an external control unit may replace the computer 20.

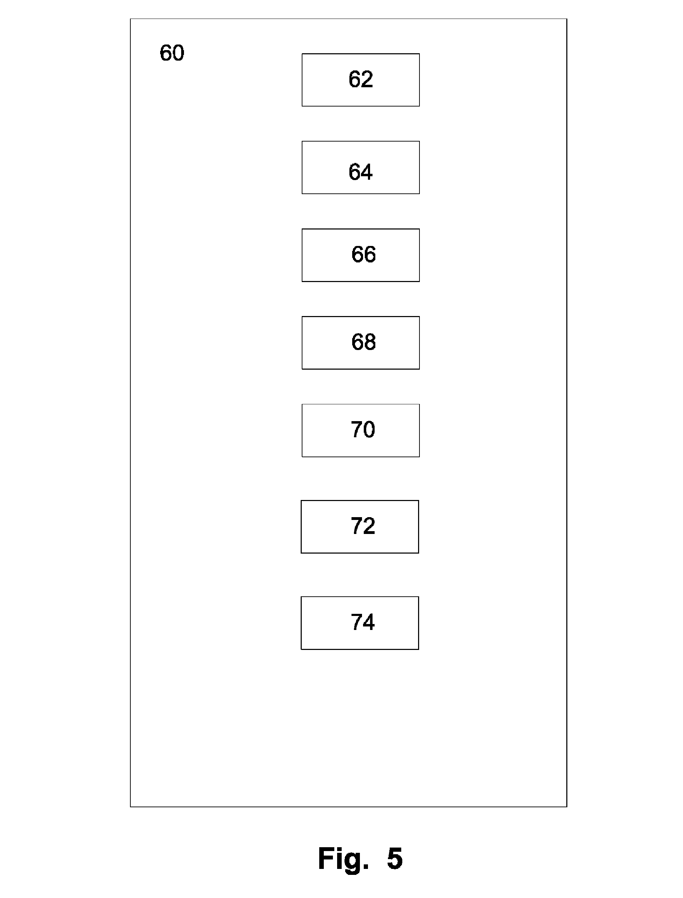

Fig. 5 shows code segments of a computer program 60 for performing diagnostics of an organ in the thoracic cavity. This computer program 60 may be embedded in a computer-readable medium and adapted to be processed by the computer 20. The computer program 60 comprises a code segment 62 for receiving at least one measurement signal. It also comprises a code segment 64 for computing a diagnosis of an organ based on the at least one measurement signal. Furthermore, it comprises a code segment 66 for searching a database for therapy methods corresponding to the diagnosis. The computer program also comprises a code segment 68 for computing a score for each therapy method according to a criterion. It also comprises a code segment 70 for selecting a therapy method with the highest score as a best matched therapy method. A code segment 72 for comparing the score of the best matched therapy method to a threshold value is also comprised. Furthermore, a code segment 74 for displaying the best matched therapy method with the corresponding score, if the score of the best matched therapy method is above the threshold value, and otherwise not displaying any therapy method is comprised.

As an alternative, the computer-readable storage medium may be non-transitory, be encoded with programming instructions and be loaded into a computerized control system of an apparatus for performing diagnostics of an organ in the thoracic cavity, whereby the programming instructions cause the computerized control unit to control the apparatus during operation.

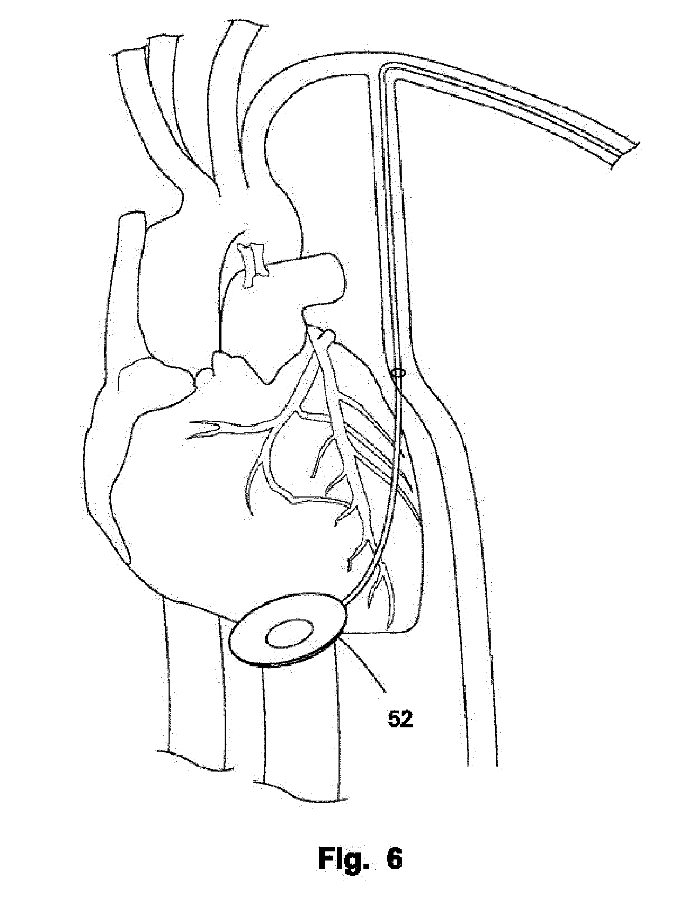

Fig. 6 is a front view of a patient and depicts a spoon-shaped tool for lifting a heart. As illustrated in Fig. 6, a tool 52, such as a spoon-shaped tool or catheter, may be used to lift the heart a shorter distance in order to reach rear sections thereof. This may be performed in order to facilitate sliding of the flexible structure 14 around the heart, so that the flexible structure will encompass a part of the heart.

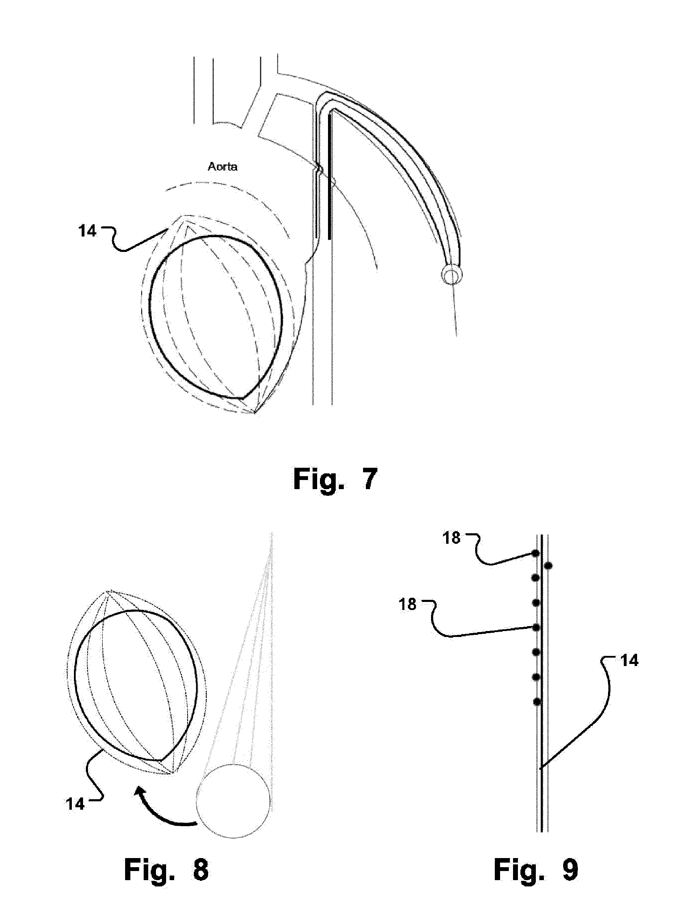

Fig. 7 depicts a step of a method of delivering a device for performing diagnostics and/or therapy of an organ in the thoracic cavity. In this method a guide wire or coronary wire is fitted to guide the flexible structure 14 from outside a body via the left internal mammary puncture from the left arm to a target site inside the body, such as in contact with, but exterior to the pericardial sack 12. The method used is called the Epicardial Access approach or Epicardial Access Surgery, and is a less invasive approach than other approaches that can be used, such as direct apical or thorax punctation. The device 1 is transluminally deliverable to the organ. A sheath or a catheter, with the device, is brought through the left arteria brachialis and further to the target site. The target site may be a site, where the device 1 is in contact with, but exterior to the pericardial sack.

Fig. 8 depicts another step of the method of delivering a device for performing diagnostics and/or therapy of an organ in the thoracic cavity. In Fig. 8, the flexible structure 14 has reached the target site just exterior to the pericardial sack. The heart may have been lifted by a tool 52 (shown in Fig. 6), such as a spoon-shaped tool or catheter. The flexible structure 14 is then slid around the heart, so as to encompass a part of the heart, including at least one apex, such as the lowest point of the heart.

Fig. 9 depicts the flexible structure 14 in its collapsed state, with transducers 18 attached to it. The flexible structure 14 is in its collapsed state only during delivery and removal. Once the flexible structure 14 has been put in its place around the heart, the flexible structure 14 will be in its expanded state until removed. Normally, the device 1 with the flexible structure 14 will be left in its expanded state for days or months.

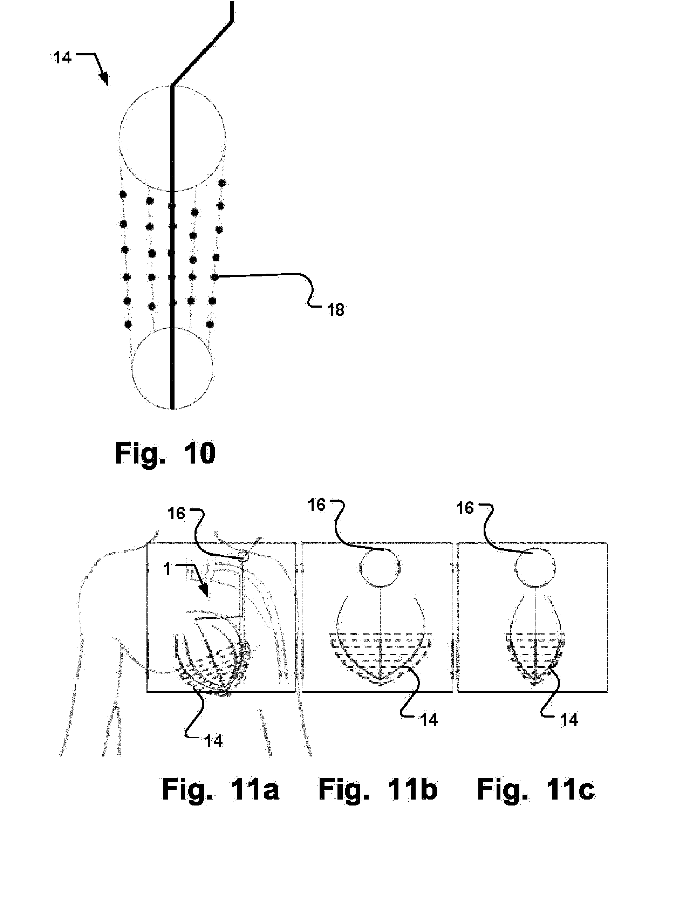

Fig. 10 depicts the flexible structure 14 in its operational state, i.e. its expanded state. In Fig. 10 the transducers 18 can be seen. The transducers 18 are attached to the flexible structure 14 and are distributed across the surface of the flexible structure 14. The transducers 18 may be a plurality of temperature sensors, accelerometers, ultrasound transmitters, ultrasound receivers, voltage sensors, potential sensors, current sensors, pH sensors, ECGECG sensors, ultrasound sensors or ablation sensors. The transducers may be distributed so as to cover the complete organ. Some of the transducers may be provided with a contact agent or displacement material, such as water or gel, since some transducers, such as ultrasound transducers, should be located a small distance away from the heart wall to be imaged. Since the transducers are distributed around the organ, pacing can be performed at any position possible. Furthermore, electro-diagnosis and therapy can be performed at any position of the heart's surface.

Fig. 11 a is a front view of a patient with a device 1. In this Figure, the flexible structure 14 is in its operational state and connected to an actuator 16. The actuator controls the shape of the flexible structure 14. By controlling the shape of the flexible structure 14, the actuator will also assist the heart in its natural contracting and/or expanding. In one embodiment, the actuator only assists the heart in contracting, whereas expansion of the heart is assisted by the flexible structure 14 and its resiliency, since the flexible structure 14 after being controlled by the actuator 16 will tend to return to its expanded state, because of its resiliency. Normally, the flexible structure 14 is made to have an expanded state as its normal state, and thus, will tend to return to this state. In another embodiment, the actuator only assists the heart in expanding, whereas contraction of the heart is assisted by the flexible structure 14 and its resiliency, since the flexible structure 14 after being controlled by the actuator 16 will tend to return to its collapsed state, because of its resiliency.

Fig. 11b shows a heart partly surrounded by a flexible structure 14, when the heart is in an expanded state. The actuator 16 is connected to the flexible structure 14 and controls the shape of it.

Fig. 11c shows a heart partly surrounded by a flexible structure 14, when the heart is in a contracted state. The actuator 16 is connected to the flexible structure 14 and controls the shape of it.

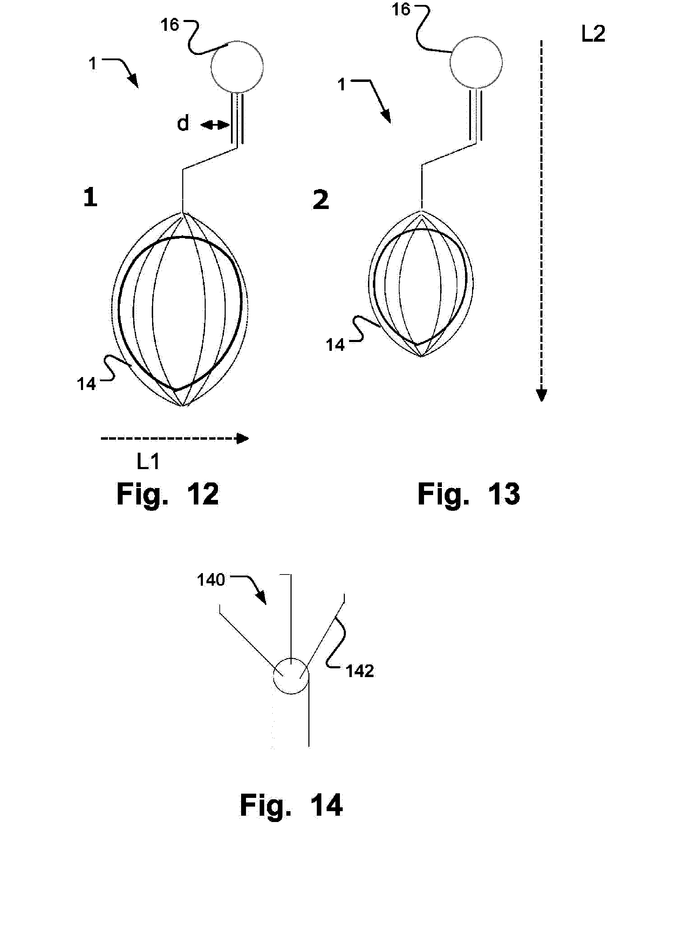

Figs. 12 and 13 show the pericardial sack substantially covered by the flexible structure 14. The exterior surface of the pericardial sack and the heart may be completely covered with the flexible structure 14. In one embodiment the flexible structure 14 is delivered as a whole unit. However, it is also possible to deliver the flexible structure 14 as two or more separate units one after another with a gap in-between. Regardless of if the flexible structure 14 is delivered as one or several units, the heart can be covered by the flexible structure quickly and through a very small access path, e.g. a path with a small diameter. The diameter d would typically be in the range of 1-20mm and preferably 2.5mm. The length *L*₁ shown in fig. 12 would typically be in the range of 1-20cm and normally approximately 7cm. The length *L*₂ shown in fig. 13 would typically be in the range of 5-25cm and normally approximately 14cm.

Fig. 14 shows a surgical tool 140 for removing a flexible structure from a heart. The tool 140 preferably has at least three arms 142. The tool may be three-arm-pliers or four-arm-pliers or any other suitable gripping tool. It is used for bringing the flexible structure 14 back to its collapsed state, so that it can be removed from the target site and the body, without causing damage to the organ or otherwise in the body.

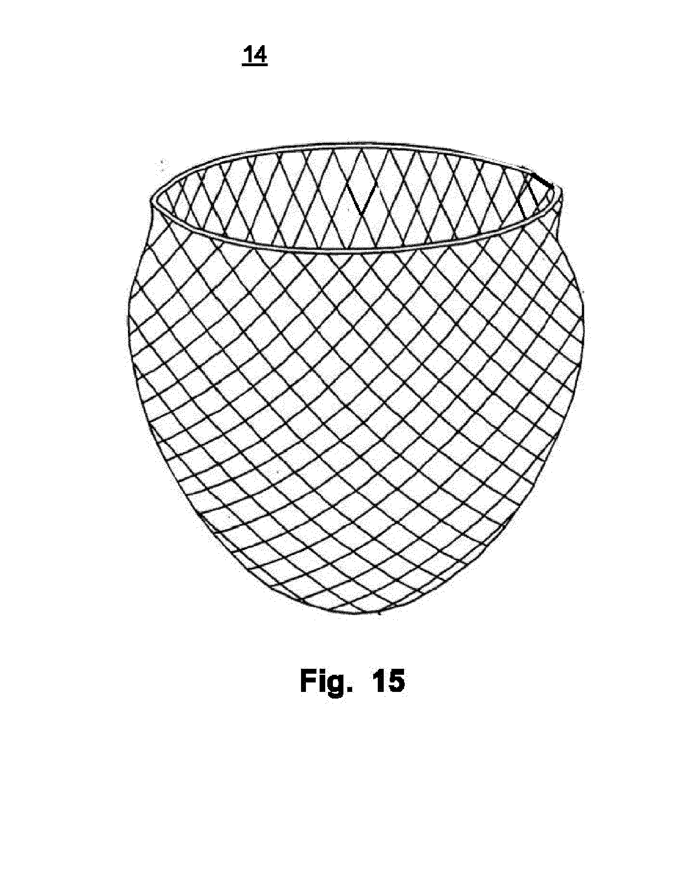

Fig. 15 shows the flexible structure 14 according to one embodiment. In this embodiment, the flexible structure 14 is a mesh.

The body is largely composed of water molecules. Each water molecule has two hydrogen nuclei or protons. When a patient is inside a powerful magnetic field of a scanner, such as an MRI scanner, the magnetic moments of some of the molecules become aligned with the direction of the field. A radio frequency transmitter, such as the internal electromagnetic coil and/or at least one other electromagnetic coil, is briefly turned on, producing a further varying electromagnetic field. The photons of this electromagnetic field at a certain frequency, known as the resonance frequency, have just the right energy to be absorbed and flip the spin of the aligned protons in the body. The protons resonate at a certain frequency, depending on the strength of the applied magnetic field. After the magnetic field is turned off, the protons which absorbed energy revert to the original lower-energy state. Now a hydrogen dipole has two spins, one high spin and one low spin. In the low spin, both the dipole and the field are in parallel direction and in the high spin case, the dipole and the field are in antiparallel direction. They release the difference in energy as a photon, and the released photons are detected by a scanner, such as the MRI scanner, as an electromagnetic signal, similar to radio waves. The scanner may use electromagnetic coils for this detection.

As a result of conservation of energy, the resonant frequency also dictates the frequency of the released photons. The photons released when the field is removed have certain energy and therefore a certain frequency, which depends on the energy absorbed, while the field was active. This relationship between the field-strength and the frequency allows the use of nuclear magnetic resonance for imaging. A MRI image can be constructed because the protons in different tissues return to their equilibrium state at different rates, which is a difference that can be detected. Five different tissue variables spin density, T1 and T2 relaxation times and flow and spectral shifts can be used to construct images. By changing the settings on the scanner, this effect is used to create contrast between different types of body tissue or between other properties, as in functional MRI (fMRI) and diffusion MRI.

In one embodiment, the internal control unit 24 is adapted to control the internal electromagnetic coil so that a varying electromagnetic field for MRI is generated. The internal control unit 24 may also be connected to at least one transducer 18 for obtaining a signal related to detected electromagnetic signals, such as electromagnetic signals emanated from photons.

In another embodiment a kit for delivery of a device is provided. The kit comprises a flexible protecting catheter for bringing the flexible structure of the removable device to a target site in a body, exterior to the pericardial sack of a heart. It also comprises a guide wire for guiding the flexible protecting catheter to the target site in the body, exterior to the pericardial sack of the heart. It further comprises a tool 52 for facilitate lifting of the heart. The kit further comprises a surgical tool for putting the flexible structure around the pericardial sack of the heart, so that the flexible structure surrounds part of the heart. Also comprised by the kit is a surgical tool, such as three-arm-pliers or four-arm-pliers, for bringing the flexible structure back to its collapsed state. The device and the whole system including kit and device are removable, i.e. they do not need to be permanently placed in a body. After therapy or inspection the flexible structure is collapsed and removed through a body opening.

In another embodiment of this disclosure, a method for delivery of a device 1 is provided. This method comprises gaining epicardial access through the use of the epicardial access approach via the left internal mammary puncture from the left arm. The method also comprises bringing the flexible structure into a collapsed state. It further comprises placing the collapsed flexible structure inside a protecting catheter. The method further comprises delivering the collapsed flexible structure with the protecting catheter to a target site exterior to the pericardial sack. Optionally, the method comprises lifting of the heart with a tool 52. Thereafter, sliding of the flexible structure around the pericardial sack of the heart, so that the flexible structure surrounds part of the heart, is performed. In some embodiments, steerable catheters can be used. These catheters can reach any epicardial position. Since the heart is accessed epicardially, the location of instruments used can be chosen more freely. Some of these instruments may be steerable. In order to steer the catheter and/or instruments, stereotaxy or a stereotaxis machine can be utilized. Stereotaxy is a minimally invasive form of surgical intervention, which makes use of a three-dimensional coordinate system to locate small targets inside the body and to perform on them some action such as ablation, biopsy, lesion, injection, stimulation, implantation, or radiosurgery (SRS).The epicardial access method has substantially lower complication prospects than conventional procedures and the patient risk is consequently substantially lower. If the epicardial access method is used, then it is also possible to perform biopsy at any epicardial position. Optionally, mediastinal biopsy, i.e. biopsy in the mediastinum, can be performed. The mediastinum lies between the right and left pleura in and near the median sagittal plane of the chest. It extends from the sternum in front to the vertebral column behind, and contains all the thoracic viscera, except the lungs.

In order to remove a device 1, the device 1 would have to be slid of the pericardial sack of the heart. Thereafter it could be brought back the same way it was delivered.

In one embodiment, a method of temporarily treatment of an organ in a thoracic cavity is provided. The method comprises providing a device 1 and activating a therapy program for an organ. In the method, activating is regulated in a control loop based on a control signal and the control signal is based on sensor measurement.

In some embodiments, the flexible structure 14 is a bag. The bag is positioned around the heart. Thereafter, the bag is filled with a fluid or a gas. The fluid or gas may be fed from a position exterior to the body. Thus, the bag can be filled up or pumped up and thereafter the bag can be emptied. This procedure can be repeated as necessary. From this action, the heart can be pumped up from a position exterior to the body, without any contact with the blood. Some embodiments of the disclosure also provide for a validation with a heart catheter, nuclear medicine, MRT, computer tomography (CT) and/or supervision by a physician.

In one embodiment, the whole system can be used for mechanical reanimation acutely and/or chronically.

In another embodiment, a small transducer or a small container, containing a plurality of transducers, is floating in the circulation of a body fluid, such as the blood. The transducers may be steered by e.g. magnets. The transducer or transducers may be sensors giving information, such as ultrasound images, temperature or pH value. One or more of the transducers may also be actuators for giving therapy.

When the organ is the heart, the device, method, kit, medium or the system can be used in conjunction with medications causing heart arrest, with a heart lung machine or with any other form of heart arrest. Furthermore, the device, the method, the kit, the medium or the system can be combined with any heart, chest wall or with lung surgery. Moreover, the device, the method, the kit, the medium or the system can be used in conjunction with organ protection solutions.

Although the methods above have been described as extra-pericardial, i.e. operating outside the pericardium, it is also possible for the methods to operate intra-pericardially mutatis mutandis.

It is also possible to perform epicardial ablation. E.g. electrical energy is applied to interrupt abnormal electrical pathways, such as Wolff-Parkinson-White (WPW) syndrome, from inside or outside the pericardial sac. Wolff-Parkinson-White syndrome (WPW) is a disorder of the heart, in which the ventricles of the heart contract prematurely due to an accessory pathway known as the bundle of Kent. This accessory pathway is an abnormal electrical communication from the atria to the ventricles.

In one embodiment, the device comprises at least one transducer 18, such as a temperature sensor, accelerometer, ultrasound transmitter, ultrasound receiver, voltage sensor, potential sensor, current sensor or pH sensor for the diagnosis, wherein the at least one transducer 18 is preferably integrated with the flexible structure 14.

In one embodiment, the device is collapsible and transluminally deliverable to the organ in a collapsed state, and operative in an expanded state, as well as removable from the organ when returned to the collapsed state.

In one embodiment, the organ is a heart and the organ support 10 is devised to be arranged exterior to a pericardial sack 12, and the flexible structure 14 is configured to cover a region of the pericardial surface of the heart, the region comprising the pericardial surface at least at an apex of the heart and the at least one actuator is adapted to actuate a movement of the flexible structure 14 in order to aid heart contractions of the heart.

In one embodiment, the organ is a lung and the organ support 10 is devised to be arranged exterior to the lung and the flexible structure 14 is configured to cover a region of the surface of the lung, the region comprising at least the surface at an apex of the lung and the at least one actuator 16 is adapted for actuating a movement of the flexible structure 14 so that the flexible structure 14 will assist lung contractions and/or expansion.

In one embodiment, the flexible structure 14 is a hollow structure, which hollow structure has the shape of a sock.

In one embodiment, the flexible structure 14 is resilient, so that the flexible structure 14 will regain its original shape after the movement of the flexible structure 14.

In one embodiment, the device comprises a plurality of ultrasound transducers 18, which are distributed on the flexible structure 14 and at least one of the ultrasound transducers 18 are used for therapy.

In one embodiment, the plurality of ultrasound transducers 18 are controlled so that at least one group of ultrasound transducers 18 are used for therapy.

In one embodiment, at least one transducer 18 is configured to provide a measurement signal for an electrical activity of a heart, and at least one electrocardiogram (ECG) is computed from the measurement signal.

In one embodiment, the device further comprises a calculating unit for computing at least one electrocardiogram so that analysis can be performed in four dimensions.

In one embodiment, at least one transducer 18 is adapted to measure alpha radiation, beta radiation and/or gamma radiation.

In one embodiment, the flexible structure 14 comprises an internal electromagnetic coil, and the internal electromagnetic coil is a transceiver of signals related to magnetic resonance imaging MRI.

In one embodiment, the device comprises: an external electromagnetic coil 22; a computer 20 for sending control signals to and/or for receiving measurement signals from the external electromagnetic coil 22, wherein the external electromagnetic coil 22 is configured to work in pair with the internal electromagnetic coil so that the signals related to magnetic resonance imaging can be conveyed via the pair and a diagnosis based on MRI can be obtained.

In one embodiment, the device further comprises an internal control unit 24 for receiving the control signals from and/or for sending the measurement signals to the internal electromagnetic coil.

In one embodiment, the internal control unit 24 is adapted to control the internal electromagnetic coil so that a varying electromagnetic field for MRI is generated

In one embodiment, the internal control unit 24 is connected to the at least one transducer 18 for obtaining a signal related to detected electromagnetic signals, such as electromagnetic signals emanated from photons.

In one embodiment, the organ support 10 is adapted to assist in diaphragmatic breathing.

In one embodiment, the assisting in diaphragmatic breathing comprises an internal movement of the whole thorax, with or without the diaphragm, so that a negative pressure breathing can be performed.

The present disclosure has been described above with reference to specific embodiments. However, other embodiments than the above described are equally possible within the scope of the disclosure. The different features and steps of the disclosure may be combined in other combinations than those described. The scope of the disclosure is only limited by the appended patent claims.

More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present disclosure is/are used.

## Claims

1. A computer-readable medium having embodied thereon a computer program (60) for processing by a computer (20), said computer program (60) performing diagnostics of an organ in the thoracic cavity and comprising:
a code segment (62) for receiving at least one measurement signal;
a code segment (64) for computing a diagnosis of an organ based on said at least one measurement signal;
a code segment (66) for searching a database for therapy methods corresponding to said diagnosis;
a code segment (68) for computing a score for each therapy method according to a criterion;
a code segment (70) for selecting a therapy method with the highest score as a best matched therapy method;
a code segment (72) for comparing said score of said best matched therapy method to a threshold value;
a code segment (74) for displaying said best matched therapy method with corresponding score if said score of said best matched therapy method is above said threshold value, and otherwise not displaying any therapy method.

2. A non-transitory computer-readable storage medium encoded with programming instructions, said storage medium being loaded into a computerized control system of an apparatus for performing diagnostics of an organ in the thoracic cavity, and said programming instructions causing said computerized control unit to control said apparatus during operation by:
receiving (80) at least one measurement signal from at least one transducer (18) at an internal control unit (24);
transmitting (82) said at least one measurement signal from said internal control unit (24) to a computer (20);
computing (84) in said computer (20) a diagnosis of an organ based on said at least one measurement signal;
searching (86) in said computer (20) a database for therapy methods corresponding to said diagnosis;
computing (88) a score for each therapy method according to a criteria;
selecting (90) a therapy method with the highest score as a best matched therapy method;
comparing (92) said score of said best matched therapy method to a threshold value; and
displaying (94) said best matched therapy method with corresponding score if said score of said best matched therapy method is above said threshold value, and otherwise not displaying (96) any therapy method.

3. A method of performing diagnostics of an organ in the thoracic cavity, comprising:
receiving (80) at least one measurement signal from at least one transducer (18) at an internal control unit (24);
transmitting (82) said at least one measurement signal from said internal control unit (24) to a computer (20);
computing (84) in said computer (20) a diagnosis of an organ based on said at least one measurement signal;
searching (86) in said computer (20) a database for therapy methods corresponding to said diagnosis;
computing (88) a score for each therapy method according to a criterion;
selecting (90) a therapy method with the highest score as a best matched therapy method;
comparing (92) said score of said best matched therapy method to a threshold value; and
displaying (94) said best matched therapy method with corresponding score if said score of said best matched therapy method is above said threshold value, and otherwise not displaying (96) any therapy method.

4. The computer program of claim 1 enabling carrying out a method according to claim 3.

5. A device for performing diagnostics and/or therapy of an organ in the thoracic cavity, comprising an organ support (10), which organ support (10) is devised to be arranged exterior to an organ, and comprises:
a flexible structure (14), such as a fabric, net, coil, bag or mesh, which is configured to cover a region of said organ, said region comprising at least the surface at an apex of said organ;
at least one actuator (16), such as a motor, for actuating a movement of said flexible structure (14) for said therapy, and
optionally at least one transducer (18).

6. The device according to claim 5, wherein said at least one actuator (16), is a motor, or at least one pneumatic and/or hydraulic actuator, such as a pump, connected to said flexible structure (14), and wherein said actuator (16) is adapted to assist a movement of said organ, such as for assisting a heart, in contracting and/or expanding.

7. The device according to claim 6, wherein said flexible structure (14) is resilient and said organ is assisted in expanding by said flexible structure (14).

8. The device according to any of claims 6-7, further comprising:
a component (40) arranged at a voltage supply (44) of said motor;
a measuring device (42), connected to said component (40) for measuring a value related to said component (40), and thereby sensing a load of said motor;
a comparison unit (46), connected to said measuring device (42) for making a comparison between said value and at least one expected value; and
a decision unit (48) for determining a condition of a heart based on said comparison.

9. The device according to any of claims 6-8, wherein said motor is provided with an internal power supply (30), comprising a battery (32), and wherein said internal power supply (30) can be recharged from an external power supply (34) through a wireless energy transfer.

10. The device according to claim 9, wherein said internal power supply (30) further comprises a radio frequency transceiver (36) and wherein said external power supply (34) comprises a radio frequency transceiver (38) and wherein said radio frequency transceivers (36, 38) are adapted to perform said wireless energy transfer.

11. The device according to claim 10, wherein said radio frequency transceivers (36, 38) also are adapted to transfer radio signals related to measurements.

12. A kit for delivery of a device according to any of claims 5-11, comprising:
a flexible protecting catheter for bringing said flexible structure of said removable device to a target site in a body, exterior and/or interior to the pericardial sack of a heart;
a guide wire for guiding said flexible protecting catheter to said target site in said body, exterior to the pericardial sack of said heart;
a tool (52) for facilitate lifting of said heart;
a surgical tool for putting said flexible structure around the pericardial sack of said heart, so that said flexible structure surrounds part of said heart; and
a surgical tool (140), such as three-arm-pliers or four-arm-pliers, for bringing said flexible structure back to its collapsed state.

13. A removable system comprising:
said kit of claim 12; and
said device of any of claims 5-11.

14. A method for delivery of a device according to any of claims 5-11, said method comprising:
gaining epicardial access through the use of the epicardial access approach via the left internal mammary puncture from the left arm;
bringing said flexible structure into a collapsed state;
placing said collapsed flexible structure inside a protecting catheter;
delivering said collapsed flexible structure with said protecting catheter to a target site exterior to the pericardial sack;
optionally lifting said heart with a tool 52; and
sliding said flexible structure around the pericardial sack of a heart, so that said flexible structure surrounds part of said heart.

15. A method of temporarily treatment of an organ in a thoracic cavity, said method comprising:
providing a device of any of claims 5-11; and activating a therapy program for an organ, wherein activating is regulated in a control loop based on a control signal based on sensor measurement.
